# EUROPÄISCHE PATENTSCHRIFT

(11) **EP 4 082 989 B1**
(45) Veröffentlichungstag und Bekanntmachung des Hinweises auf die Patenterteilung: **22.01.2025**
(21) Anmeldenummer: 22170273.1
(22) Anmeldetag: 27.04.2022
(51) Int. Cl.: C04B 28/14, C04B 40/00, C04B 24/14, A47F 10/04, E04H 3/02

(54) **HYDROPHOBIERENDER MÖRTELCOMPOUND**
HYDROPHOBING MORTAR COMPOUND
COMPOSÉ HYDROPHOBE DE MORTIER

(30) Priorität: 30.04.2021 DE 202021001595 U
(43) Veröffentlichungstag der Anmeldung: 02.11.2022
(73) Patentinhaber: IAB - Institut für Angewandte Bauforschung Weimar gemeinnützige GmbH, 99428 Weimar (DE)
(72) Erfinder: Piethe, Vivienne, 99423 Weimar (DE)
(74) Vertreter: Gleim Petri Patent- und Rechtsanwaltspartnerschaft mbB

(56) Entgegenhaltungen:
- EP-A1- 0 051 150
- CN-A- 111 732 399
- GB-A- 1 040 842
- US-A- 1 048 695
- US-A- 1 658 605

## Beschreibung

### Technisches Gebiet der Erfindung

Die vorliegende Erfindung betrifft ein Additiv für gipsbasierte BindemittelSysteme zur Reduktion der Wasseraufnahme in abgebundenen Systemen.

### Stand der Technik

Gipsbasierte Bindemittel wurden ab dem frühen Mittelalter vorwiegend im Mörtel- oder Estrichbereich eingesetzt. Der Anwendungsbereich beschränkte sich im Gegensatz zu heute nicht nur auf den Innenbereich, sondern umfasste auch den Einsatz in ausgesetzten Mauerwerken. Viele historische Bestandsbauten aus Calciumsulfatbasierten Baustoffen in Deutschland befinden sich in unmittelbarer Nähe der Gipslagerstätten, wie dem Harz, dem Thüringer Becken, Franken und der Umgebung der Kalkberge beim niedersächsischen Lüneburg und dem schleswig-holsteinischen Bad Segeberg. Dies ist beispielsweise beschrieben in: Tesch, V: Gefügeoptimierte Instandsetzungsmörtel auf Calciumsulfat-Basis für die Anwendung im Außenbereich, Schriftenreihe Baustoffe und Massivbau Heft 6, Dissertation, Kassel, 2006*.*

Besonders kritisch beim Einsatz von Gips-Bindemitteln im Bauwesen ist die hohe Löslichkeit von 2,14 g/I (bei 20°C) im Vergleich mit anderen mineralischen Baustoffen (so beschrieben in: *https:*//*www.salzwiki.de*/*index.php*/*Gips, aufgerufen 07.01.2021).* Diese Eigenschaft schließt die Anwendung von Gips-Bindemitteln im Außenbereich eigentlich aus, aber historische Gipsmörtel weisen ungeachtet dessen eine enorme Dauerhaftigkeit auf, die die der heutigen Mörtelsysteme übertreffen. Leider sind heute keine Dokumentationen über die Herstellung und Rezepturen der historischen Systeme überliefert. Es wurden im Rahmen einer Dissertation lediglich vergleichende REM-Aufnahmen gefertigt. Diese zeigen eine deutliche Differenz in der Gefügeausbildung der historischen zu den aktuellen Gipsmörteln. Der historische Mörtel weist viel gröbere Gipskristalle auf als der heutige Mörtel auf.

Trotz der höheren Dauerhaftigkeit müssen auch die historischen Gipsbindemittel im Laufe der Zeit Instand gesetzt werden, da durch Fehlstellen und Risse verstärkt Feuchtigkeit ins Mauerwerk eindringen kann. Dies führt zu vermehrten Löseerscheinungen, sodass es schnell zur Gefährdung der Standsicherheit kommen kann.

Während des vergangenen Jahrhunderts war der Reaktionsmechanismus der nachträglichen Ettringitbildung noch unbekannt, sodass standardmäßig zementäre Bindemittelsysteme in gipshaltige Mauerwerke injiziert wurden. Diese Ettringitbildung lasst sich wie folgt chemisch beschreiben:

3CaO • Al₂O₃ • 6H₂O + 20H₂O + 3(CaSO₄ • 2H₂O) → 3CaO • Al₂O₃ • 3CaSO₄ • 32H₂O Calciumaluminathydrat (Zement) + Wasser + Gips → Ettringit

Diese Reaktion geht mit einer Volumenzunahme um rund 4,8 % einher, welche das Mauerwerk nachhaltig schädigt, anstatt es instand zu setzen. Wurden die Systeme vollflächig in das Bestandsgebäude eingebracht, ist nicht nur die Standsicherheit gefährdet, sondern in den meisten Fällen ist das Gebäude kaum noch und nur unter enormen Kosten- und Zeitaufwand zu sanieren. Ein bekanntes Beispiel für eine falsche Instandsetzung ist die Runneburg in Weißensee (Thüringen, Deutschland). Der Wohnturm musste aufgrund der Folgen der nachträglichen Ettringitbildung mithilfe eines Stahlkorsetts gesichert werden (nachzulesen in: Eckart, A.: Befunde/Schäden an historischen Baukonstruktionen Naturwände/historische Ziegel, Vorlesungsskript, Weimar, 2017*).*

Neben der Bildung von Ettringit kann auch Thaumasit bei gleichzeitigem Vorhandensein von Zement und Gips entstehen. Problematischer ist diese Reaktion allerdings in einem zementären Baustoffsystem, da die festigkeitsbildenden C-S-H-Phasen des Zementsteins zersetzt werden, sodass das gesamte Bauteil entfestigt werden kann und die Standsicherheit enorm gefährdet werden kann. Dennoch ist dieses Phänomen bei der Sanierung von gipshaltigem Mauerwerk ebenfalls zu berücksichtigen, vor allem wenn in der Vergangenheit mit inkompatiblen zementären Bindemitteln saniert wurde. Thaumasit entsteht bevorzugt bei Temperaturen unterhalb von 15 °C, wohingegen Ettringit bei Temperaturen bis zu 70 °C stabil bleibt. Neben Zement und Gips sind auch carbonatische Reaktionspartner für die Bildung von Thaumasit notwendig (nachzulesen in: Eckart, A.: Befunde/Schäden an historischen Baukonstruktionen Naturwände/historische Ziegel, Vorlesungsskript, Weimar, 2017*):*

3CaO • 2SiO₂ • 3H₂O + 24H₂O + 2CaSO₄ • 2H₂O + 2CaCO₃ →

2(CaSiO₃ • CaSO₄ • CaCO₃) • 15H₂O + Ca(OH)₂

C-S-H-Phase (Zement) + Wasser + Gips + Kalk → Thaumasit

Genaue Angaben über die Anzahl von historischen Gebäuden aus Gipsmauerwerk ist derzeit nicht verfügbar. Allerdings gibt es in Deutschland ca. 1,3 Millionen Baudenkmäler, von denen schätzungsweise mindestens ein Drittel gefährdet oder sanierungsbedürftig sind. Vermutlich bestehen davon etwa 10% aus Gips (diese Angabe basiert auf dem flächenbezogenen deutschlandweiten Gipsvorkommen). Somit kann angenommen werden, dass etwa 130.000 Baudenkmäler in Deutschland aus Gips bestehen. Hinzu kommen weitere historische Gebäude, die nicht in den Denkmalschutzbereich fallen. Anzunehmen wäre, dass sich die Summe aller historischen Gebäude aus Gipsmauerwerk in Deutschland auf ca. 250.000 bis 400.000 beläuft.

Um die Dauerhaftigkeit und Verwitterungsresistenz von Gipsmörteln und Stuckgipsen zu erhöhen, sind im Stand der Technik verschiedene Methoden bekannt.

Bisherige Lösungen beruhen einerseits auf einem nachträglichen oberflächlichen Auftrag von Hydrophobierungsmitteln. In WO 95/25706 ist somit ein Verfahren zur Behandlung von mit mineralischen Materialien errichteten Bauten beschrieben, auf die eine hydrophobierende Substanz auf der Basis von Silanan und / oder Siloxanen aufgetragen wird.

In EP 2 240419 ist ein Gemisch aus einem mineralischen Bindemittel auf Basis von Calciumsulfat und mindestens einem Additiv, das dem mineralischen Bindemittel auf Basis von Calciumsulfat hydrophobe Eigenschaften verleiht. Dieses Bindemittelgemisch enthält ein mineralisches Bindemittel auf Basis von Calciumsulfat und mindestens ein Gemisch aus Fettsäuresalzen von mindestens zwei verschiedenen Metallkationen oder zwei verschiedenen Ammoniumkationen oder einem Gemisch aus mindestens einem Metallkation und mindestens einem Ammoniumkation, wobei die Metallkationen aus der Gruppe bestehend aus Alkalimetallen, Erdalkalimetallen, Zink, Aluminium und seltenen Erden ausgewählt werden und wobei der Anteil der Fettsäuresalze von Fettsäuren mit 8 bis 17 C-Atomen, bezogen auf die Gesamtmenge der Fettsäuresalze im Fettsäuresalzgemisch, 20 Gew.-% oder mehr als 20 Gew.-% oder der Anteil an Fettsäuren mit 8 bis 14 C-Atomen 10 Gew.-% oder mehr als 10 Gew.-%, bezogen auf die Gesamtmenge der Fettsäuresalze im Fettsäuresalzgemisch, oder beides, ausmacht. Bei erfindungsgemäßen Baumaterialien, die aus einem erfindungsgemäßen Bindemittelgemisch erhältlich sind, kann es sich beispielsweise um Gipsplatten, Gipsblöcke oder sonstige Formteile, insbesondere Gipskartonplatten handeln.

Eine Baustoffmasse, die mindestens ein hydrophobierendes Pulver enthält ist in DE 100 49 072 beschrieben, wobei das Pulver mindestens einen Carbonsäurerest enthält.

In DE 195 42 443 C2, DE 100 49 127 C2 und DE 44 02 408 A1 wird ein Verfahren zur Kunststoffvergütung von mineralischen Beschichtungsmittel- und BindemittelZusammensetzungen mittels in Wasser redispergierbarem Pulver beschrieben. In einer beschriebenen Ausführungsform wird einer Dispersionspulverzusammensetzung ein Teil an Schutzkolloidmenge zugegeben, wobei als geeignetes Schutzkolloid Casein genannt wird.

Der Einsatz von Lycopodium in Verbindung mit Calciumsulfat ist aus DE 101 33 399 A1 bekannt, wobei spezielle Zusammensetzungen auf Wachsbasis angegeben sind, die die Grundlage für kosmetische Mittel bilden können und insbesondere zur Imprägnierung und Benetzung von Gebrauchs- und Hygienetüchern für die Körperreinigung und -pflege eingesetzt werden können.

Ein pulverförmiges Oberflächenschutzsystem wird in DE 102 39 071 A1 beschrieben, dass auf Flächen wie Kunststofffolien, Metall, lackiertes Metall, Keramik, Papier, Leder, Holz, aber auch Fasern und Garne, sowie Baustoffe wie Mauerwerk, Beton, Ziegel, Gipsflächen und dergleichen aufgebracht werden kann. Das Pulver kann anorganischer oder organischer Natur sein. Beispiele für geeignete teilchenförmige Materialien sind organische Pulver, z.B. Polymerpulver wie Polyethylenpulver, Polypropylenpulver, Polytetrafluorethylenpulver, und bioorganische Pulver wie Lycopodium. Anorganische Pulver können beispielsweise solche auf Basis von Siliziumdioxid oder Silikaten wie Kieselsäurepulver, z.B. Fällungskieselsäure, Diatomeen-Erde, pyrogene Kieselsäure, Porosile und Kieselgelpulver, Tonmineralien, Quarzpulver, Glaspulver, z.B. Glaskugeln, weiterhin Aluminiumoxid, Zeolithe, Titandioxid, wobei oxidische Pulver auch mit fluororganischen Silanen hydrophobiert sein können. Der Auftrag der Pulver kann mittels Haftkleber und anschließendem Aufstreuen und Abblasen oder durch fotovernetzbaren Klebstoff oder einen härtbaren Lack erfolgen.

Zusammensetzungen und Verfahren zum Wasserdichtmachen von feuchten Strukturen aus wasserdurchlässigen oder -korrodierbaren Baumaterialien wird in DE 689 23 137 T2 beschrieben, wobei die Grundzusammensetzung C8- bis C18-Fettsäuren enthält.

Ein Zusatzmittel für mineralische Bindemittelzusammensetzungen, insbesondere Mörtel oder Beton zur Verbesserung der Frost-Beständigkeit und der Frost-Tausalz-Beständigkeit wird in WO 2021/037996 beschrieben, bei der ein Reduktionsmittels, ein Verdickungsmittel, ein Luftporenbildner und mindestens ein Lösemittel eingesetzt werden. Das Verdickungsmittel kann dabei beispielsweise Gelatine und / oder Casein sein.

In WO 2007/036324 ist ein in Wasser redispergierbares Additiv beschrieben, das die Reduktion von Ausblühungen in hydraulisch abgebundenen Systemen betrifft, dass insbesondere als Additiv für die Zugabe bei Trockenmörteln geeignet ist. Das in Wasser redispergierbare Pulver besteht aus mindestens einer organischen Komponente und mindestens eines wasserlöslichen organischen polymeren Schutzkolloids sowie gegebenenfalls weiteren Additiven, wobei die organische Komponente mindestens eine Verbindung mit einer cyclischen Gruppe enthält, die ganz oder teilweise gesättigt ist, und einen Schmelzpunkt von etwa -20 °C bis 250 °C sowie ein Molekulargewicht von etwa 100 bis 10.000 besitzt, und mit dem wasserlöslichen organischen polymeren Schutzkolloid in Wasser eine stabile Dispersion bildet, wobei das Gewichtsverhältnis der organischen Komponente zum wasserlöslichen organischen polymeren Schutzkolloid etwa 95:5 bis 5:95 beträgt. Das erfindungsgemäße Additiv kann in hydraulisch abbindenden Massen insbesondere in Beton, Gips- und / oder Kalk- und / oder Zement-Putzen, Reparatur- und / oder Vollwärmeschutzmörtel, Fugen und/ oder Fliesenklebern, Nivellier- und / oder Spachtelmassen, Dichtungsschlämmen und/ oder als Additiv für Betonanstriche sowie für Klebstoffe verwendet werden.

In WO 2006/058655 wird ein hydrophobierendes Additiv in Form eines in Wasser redispergierbaren Pulvers oder einer wässrigen Dispersion beschrieben, die ein oder mehrere wasserunlösliche, filmbildende Basispolymerisate auf der Basis von Homo- oder Mischpolymerisaten von einem oder mehreren Monomeren aus der Gruppe umfassend Vinylester von unverzweigten oder verzweigten Alkylcarbonsäuren mit 1 bis 15 C-Atomen besteht.

Diesen Lösungen haftet der Nachteil an, dass Hydrophobierungsmittel auf Siloxan-/Silan-Basis meist für zementäre Systeme geeignet sind, da in Calciumsulfatbasierenden Systemen die Silizium-Komponente für deren Wirksamkeit bzw. Vernetzung fehlt. Durch den nachträglichen Auftrag von Silanen auf die fertige Oberflächenstruktur wird ein dichter Film auf der Bauteiloberfläche erzielt.

Andere hydrophobierte Gips-Bindemittelsysteme basieren auf dem Einsatz von Fettsäuresalzen, Carbonsäure-haltigen Pulvern oder Zellulosefasern. Die Wirksamkeit dieser Produkte reicht lediglich für den Gebrauch im Innenbereich aus. Carbonsäurehaltige Pulver neigen zur Hydrolyse (Spaltung durch Wasser), so dass die Wirksamkeit der Hydrophobierung nach Stattfinden dieser Reaktion deutlich an Wirksamkeit verliert. Der Einsatz von Zellulosefasern beeinträchtigt hingegen die Verarbeitbarkeit des Bindemittels enorm, so dass entweder der Wasser/Bindemittel-Wert deutlich erhöht werden muss, was wiederum zu einer Abnahme der Druck- und Dauerhaftigkeit führt, oder es muss mit weiteren Zusatzmitteln gegengesteuert werden. Der Einsatz von Casein ist ebenfalls Stand der Technik, wobei es meist im Bereich von 30% bis 50% vom Feststoffgehalt zugegeben wird. Es bewirkt, dass vermehr lange nadelförmige Gips-Kristalle (15-30 µm) ausgebildet werden, welche aufgrund ihrer Größe zwar eine höhere Wasserbeständigkeit aufweisen, aber dennoch nur für den Innenbereich geeignet sind.

Das Dokument US 1 048 695 A offenbart eine wasserabweisende Zusammensetzung für Gips, umfassend chinesisches Holzöl, Calciumstearat, Casein, ein Konservierungsmittel dafür und Wasser.

### Beschreibung der Erfindung

Die Aufgabe der Erfindung ist es daher, einen hydrophobierenden Calciumsulfat-Mörtel-Compound auf Basis von Bioadditiven bereitzustellen, der die Dauerhaftigkeit und Verwitterungsresistenz von Gipsmörteln und Stuckgipsen weiter verbessert.

Diese Aufgabe wird durch ein Hydrophobierungsmittel für Calciumsulfat-Bindemittel und calciumsulfathaltige Bindemittel mit den Merkmalen des Anspruchs 1 gelöst. Vorteilhafte Ausgestaltungen sind in den Unteransprüchen beschrieben.

Dabei werden Bioadditive aus der Gruppe der Lycopodien, wie beispielsweise Bärlappsporen in Kombination mit Casein eingesetzt, die eine interne Hydrophobierung erzielen. Durch diese Funktionalisierung des Mörtels wird der Einsatz im Außenbereich und zur Instandsetzung von gipshaltigen Bestandsgebäuden ermöglicht.

Der Keulen-Bärlapp (Lycopodium clavatum) ist eine Gefäßsporenpflanze und vor allem in Nord- und Mitteleuropa heimisch und in Amerika, Asien und Russland verbreitet. Die Sporen der Pflanze sind rund 34 µm groß und weisen ein sehr hohes A/V-Verhältnis (Verhältnis von Hüllfläche A und Volumen V) auf, wodurch sie sehr leicht entflammbar sind und für Effekte der Pyrotechnik eingesetzt werden. Darüber hinaus sind sie aufgrund ihres Aufbaus und ihres hohen Ölgehalts sehr hydrophob, sodass sie in der Pharmazie als Überzug von Tabletten verwendet werden, um ein Verkleben von z.B. Tabletten untereinander zu verhindern. Bärlappsporen weisen Strukturen im mikrofeinen Bereich auf, so dass ein sogenannter "Lotus-Effekt" erzeugt wird. Diese Struktur können die Wassermoleküle nicht durchdringen. Die Oberflächenenergie des Baustoffs wird herabgesetzt. Wasser besitzt allerdings eine sehr hohe Oberflächenspannung, wodurch diese Moleküle abgestoßen und nicht mehr angezogen werden. Übertragen auf den Baustoff bedeutete es, dass eine Flüssigkeit nicht mehr ins Innere eindringen kann, da die Oberflächenenergie des Feststoffes herabgesetzt wird. Da Wasser eine sehr hohe Oberflächenspannung besitzt, wird Wasser durch den Einsatz dieser Art der Hydrophobierung nicht mehr angezogen, sondern abgestoßen.

Das Casein trägt auf zweifache Weise zur erfindungsgemäßen Hydrophobierung bei. Zum einen fungieren die Partikel als Füllstoff im abgebundenen Gipsmörtelsystem. Hohlräume zwischen den Calciumsulfat-Dihydrat-Kristalliten werden durch das Casein gefüllt, sodass Feuchtigkeit nur noch erschwert in den Mörtel eindringen kann, da die Gesamtporosität herabgesetzt wird. Zum anderen lagert sich das Casein an die Calciumsulfat-Kristalle an. Dies sorgt für einen verzögernden Effekt, da das Wasser, welches für das Abbinden mit den Calciumsulfat-Kristalliten zu Calciumsulfat-Dihydrat reagiert, schwerer an die Kristalle "andocken" kann. Dies hat zur Folge, dass anstelle vieler kleiner Kristalle wenige große Kristalle entstehen, die im Vergleich zu den kleineren Kristallen eine geringere Löslichkeit besitzen. Die Dauerhaftigkeit des erhärteten Mörtels wird somit deutlich erhöht.

Durch die Kombination aus Bärlappsporen und Casein wird das Mörtelsystem mithilfe unterschiedlicher Reaktionsmechanismen hydrophobiert. Der Hydrophobierungseffekt durch die Verwendung beider nachhaltigen Naturprodukte übertrifft den bei der Verwendung der Komponenten im Einzelnen bei weitem.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

### Ausführungsbeispiele

Die Ergebnisse der folgenden Beispiele wurden mittels Prüfverfahren zum Messen des Kontaktwinkels für die Bestimmung der freien Oberflächenenergie einer festen Oberfläche nach DIN EN ISO 19403-2:2020 ermittelt. Bei diesem Verfahren werden mindestens drei Tropfen von mindestens zwei Prüfflüssigkeiten auf einer ebenen Probenkörperoberfläche dosiert. Aus den gemittelten Kontaktwinkeln jeder Flüssigkeit, ihren Oberflächenspannungen und deren polaren und dispersen Anteilen, wird die freie Oberflächenenergie des Festkörpers mit einem geeigneten Modell berechnet. Das Ergebnis wird als Kontaktwinkel angegeben, siehe dazu auch: *DIN EN ISO 19403-2: Beschichtungsstoffe - Benetzbarkeit - Teil 2: Bestimmung der freien Oberflächenenergie fester Oberflächen durch Messung des Kontaktwinkels (ISO 19403-2:2017); Deutsche Fassung EN ISO 19403-2:2020, April 2020.*

Für ein erstes Ausführungsbeispiel wurde an einer Stuckgips-Zusammensetzung (nur Beta-Halbhydrat) der Kontaktwinkel nach dem oben beschriebenen Verfahren für verschiedene Verhältnisse von Bärlappsporen und Casein in der Zusammensetzung des Hydrophobierungsmittels ermittelt:

| Zusammensetzung | Kontaktwinkel |
|---|---|
| Referenz (Beta-Halbhydrat) | 33,2° |
| 0,1 M % Bärlappsporen + 0,1 M % Casein | 40,1° |
| 0,4 M % Bärlappsporen + 0,4 M % Casein | 79,3° |
| 1,2 M % Bärlappsporen + 1,2 M % Casein | 95,2° |

Für ein zweites Ausführungsbeispiel wurde an einem historischen Gipsmörtel der Kontaktwinkel nach dem oben beschriebenen Verfahren für verschiedene Verhältnisse von Bärlappsporen und Casein in der Zusammensetzung des Hydrophobierungsmittels ermittelt:

| Zusammensetzung | Kontaktwinkel |
|---|---|
| Referenz (historischer Gipsmörtel) | nicht messbar |
| 0,1 M % Bärlappsporen + 0,1 M % Casein | 79,3° |
| 1,2 M % Bärlappsporen + 1,2 M % Casein | 112,6° |

In beiden Ausführungsbeispielen bezieht sich die Angabe "M %" auf Masseanteile vom trockenen Bindemittel.

## Patentansprüche

1. Hydrophobierungsmittel für Calciumsulfat- und calciumsulfathaltige Bindemittel enthaltend a) Sporen von Pflanzen aus der Familie der Bärlappgewächse (Lycopodiaceae) und b) Casein.

2. Hydrophobierungsmittel nach Anspruch 1, **dadurch gekennzeichnet, dass** die Sporen von Pflanzen aus der Familie der Bärlappgewächse Sporen von Bärlappe (Lycopodium) oder / und Sporen des Keulen-Bärlapp (Lycopodium clavatum) sind

3. Hydrophobierungsmittel nach Anspruch 1, **dadurch gekennzeichnet, dass** die Sporen von Pflanzen aus der Familie der Bärlappgewächse eine Mischung verschiedener Sporen aus der Familie der Bärlappgewächse sind.

4. Hydrophobierungsmittel nach einem der Ansprüche 1 bis 3, enthaltend zwischen 0,1 M % und 1,2 M % Bärlappsporen und/oder zwischen 0,1 M % und 1,2 M % Casein.

5. Hydrophobierungsmittel nach Anspruch 4, **dadurch gekennzeichnet, dass** die Massenanteile von Bärlappsporen und Casein gleich sind.

6. Verfahren zur Herstellung einer Zusammensetzung eines Calcuimsulfat-Bindemittels oder eines calciumsulfathaltigen Bindemittels und eines Hydrophobierungsmittels, welches Sporen von Pflanzen aus der Familie der Bärlappgewächse (Lycopodiaceae) und Casein enthält, bei dem das Hydrophobierungsmittel entweder beim Mischen von trockenen Ausgangsstoffen des Bindemittels zugegeben wird oder beim Vermengen von Ausgangsstoffen des Bindemittels mit Anmachwasser.

7. Verwendung eines Hydrophobierungsmittels nach einem der Ansprüche 1 bis 5 als Bestandteil von Trockenmischungen, bevorzugt in Sack- oder Siloware.

## Claims

1. A hydrophobizing agent for calcium sulfate and calcium sulfate-containing binders comprising a) spores of plants from the family Lycopodiaceae, and b) casein.

2. The hydrophobizing agent according to claim 1, **characterized in that** the spores of plants from the family Lycopodiaceae are spores of clubmoss (Lycopodium) and/or spores of common clubmoss (Lycopodium clavatum).

3. The hydrophobizing agent according to claim 1, **characterized in that** the spores of plants from the family Lycopodiaceae are a mixture of different spores from the family Lycopodiaceae.

4. The hydrophobizing agent according to any one of claims 1 to 3, comprising between 0.1 wt.% and 1.2 wt.% clubmoss spores and/or between 0.1 wt.% and 1.2 wt.% casein.

5. The hydrophobizing agent according to claim 4, **characterized in that** the mass fractions of clubmoss spores and casein are equal.

6. A method for producing a composition of a calcium sulfate binder or a calcium sulfate-containing binder and a hydrophobizing agent, which contains spores of plants from the family Lycopodiaceae and casein, wherein the hydrophobizing agent is added either during the mixing of the dry raw materials of the binder or during the combining of the raw materials of the binder with mixing water.

7. Use of a hydrophobizing agent according to any one of claims 1 to 5 as a component of dry mixtures, preferably in bagged or silo-stored goods.

## Revendications

1. Agents hydrophobes pour liants à base de sulfate de calcium et contenant du sulfate de calcium, comprenant a) des spores de plantes de la famille des Lycopodiacées (Lycopodiaceae), et b) des caséines.

2. Agents hydrophobes selon la revendication 1, **caractérisés en ce que** les spores de plantes de la famille des Lycopodiacées sont des spores de Lycopodes (Lycopodium) et/ou des spores de Lycopode en massue (Lycopodium clavatum).

3. Agents hydrophobes selon la revendication 1, **caractérisés en ce que** les spores de plantes de la famille des Lycopodiacées sont un mélange de différentes spores issues de la famille des Lycopodiacées.

4. Agents hydrophobes selon l'une des revendications 1 à 3, contenant entre 0,1 % en masse et 1,2 % en masse de spores de Lycopode et/ou entre 0,1 % en masse et 1,2 % en masse de caséine.

5. Agents hydrophobes selon la revendication 4, **caractérisés en ce que** les proportions en masse des spores de Lycopode et de la caséine sont égales.

6. Procédé de fabrication d'une composition d'un liant à base de sulfate de calcium ou d'un liant contenant du sulfate de calcium et un agent hydrophobe, lequel contient des spores de plantes de la famille des Lycopodiacées (Lycopodiaceae) et des caséines, dans lequel l'agent hydrophobe est ajouté soit lors du mélange des matières premières sèches du liant, soit lors du mélange des matières premières du liant avec l'eau de gâchage.

7. Utilisation d'un agent hydrophobe selon l'une des revendications 1 à 5 en tant que composant de mélanges secs, de préférence dans des sacs ou des silos.
